# EUROPÄISCHE PATENTSCHRIFT

(11) **EP 0 482 320 B1**
(45) Veröffentlichungstag und Bekanntmachung des Hinweises auf die Patenterteilung: **11.09.1996**
(21) Anmeldenummer: 91114637.1
(22) Anmeldetag: 30.08.1991
(51) Int. Cl.: A61F 2/34

(54) **Schalenelement zur Aufnahme einer Gelenkendoprothese**
Acetubular cup for receiving a joint endoprosthesis
Coque acétabulaire pour la réception d'une endoprothèse d'articulation

(30) Priorität: 20.10.1990 DE 9014542 U
(43) Veröffentlichungstag der Anmeldung: 29.04.1992
(73) Patentinhaber: Howmedica GmbH, D-24232 Schönkirchen (DE)
(72) Erfinder: Täger, Karl Heinrich, Prof. Dr. med., W-8035 Gauting (DE)
(74) Vertreter: Patentanwälte Hauck, Graalfs, Wehnert, Döring, Siemons

(56) Entgegenhaltungen:
- EP-A- 0 179 736
- EP-A- 0 214 885
- EP-A- 0 329 019
- DE-U- 8 710 796
- DE-U- 9 014 542
- FR-A- 2 617 040
- FR-A- 2 622 432

## Beschreibung

Die Erfindung bezieht sich auf ein Schalenelement zur Aufnahme einer Hüftgelenkendoprothese nach dem Oberbegriff des Anspruchs 1, wie es beispielsweise aus der EP-A-0 329 019 als bekannt hervorgeht.

Endoprothesen für den Ersatz des Hüftgelenks sind seit langer Zeit bekannt. Für die Befestigung der Gelenkkugel der Endoprothese in dem Hüftknochen werden Gelenkpfannen verwendet. Aus der DE-A-23 01 801 ist bekannt, die Gelenkpfanne aus einer Außen- und Innenkappe herzustellen, wobei die lösbar in der Außenkappe untergebrachte Innenkappe die Gelenkkugel aufnimmt. Aufgrund der ständigen, einseitigen Belastung durch die Kugel kommt es leicht zu Lockerungen der Gelenkpfanne. Die EP-A-0 303 006 offenbart, die Außenfläche des Pfannenkörpers zur Verbesserung des Verwachsens der Prothese mit dem Knochenmaterial zu verkugeln. Zur weiteren Verbesserung der Prothesenbefestigung am Knochen wird in der EP-A-0 329 019 vorgeschlagen, ein aus Außen- und Innenschale bestehendes, einschraubbares Schalenelement zu verwenden, bei dem runde oder langgestreckte Durchbrüche in der Außenschale vorgesehen sind. Vor Einsetzen des Schalenelementes in den Hüftknochen kann über diese Öffnungen Knochenmaterial in den zwischen Außen- und Innenschale ausgebildeten Zwischenraum eingefügt werden. Nach Implantation der Prothese kann körpereigenes Hüftknochenmaterial durch die Öffnungen mit dem eingebrachten Knochenmaterial verwachsen und so die Prothese fester mit dem Knochen verbinden. Zum Einschrauben des Schalenelementes sind auf der Außenseite der Außenschale Gewindegangabschnitte geformt, die an Nuten enden, die mit den Gewindeabschnitten ein Schneidgewinde bilden.

Aus dem Dargestellten ergibt sich, daß für ein Einschrauben des Schalenelementes ein selbstschneidendes Gewinde wichtig ist, und daß andererseits der Grad der Verwachsung des Hüftknochens mit dem Schalenelement für eine sichere und nicht wieder lösbare Befestigung des Schalenelementes und damit der Gelenkendoprothese an dem Knochen wesentlich ist.

Der Erfindung liegt daher die Aufgabe zugrunde, ein Schalenelement zu schaffen, bei dem das Einschrauben in die Knochenhöhlung und die wirksame Verwachsung mit den Knochen zwecks langdauernder fester Verbindung mit den Knochen noch verbessert ist.

Diese Aufgabe wird gelöst durch die Merkmale des Kennzeichnungsteils des Anspruchs 1.

Bei dem erfindungsgemäßen Schalenelement sind im wesentlichen quer zur Umfangsrichtung und nahezu über die Höhe der Außenschale und über die gesamte Nutbreite sich erstreckende, längliche Öffnungen vorgesehen, die zugleich als Nuten eines Schneidgewindes fungieren. Durch diese Anordnung der Öffnungen zwischen den sich im wesentlichen längs zur Umfangsrichtung erstreckenden Gewindegangabschnitten ist nicht nur gewährleistet, daß vor dem Einschrauben des Schalenelementes auf einfache Weise Knochensubstanz in den zwischen Innen- und Außenschale gebildeten Zwischenraum eingebracht werden kann. In vorteilhafter Weise kann darüber hinaus das beim Einschrauben des Schalenelementes abgetragene Knochenmaterial über die länglichen Öffnungen zwischen den Gewindegangabschnitten dem Zwischenraum zugeführt werden. Ein Herausfallen der in den Zwischenraum eingebrachten Knochensubstanz wird während der Operation verhindert.

Mit der erfindungsgemäßen Anordnung der Öffnungen ist es insbesondere möglich, diesen eine optimale Längserstreckung zu geben, nämlich nahezu über die gesamte Höhe der Außenschale, so daß nach erfolgter Implantation die Verwachsung des Knochens mit dem Schalenelement über eine relativ große Fläche erfolgen kann und somit das Schalenelement am Knochen sicher und dauerhaft befestigt ist.

Nach einer bevorzugten Ausgestaltung der Erfindung ist das Schneidgewinde doppelgängig ausgebildet, wodurch sich ein zusätzlicher positiver Halteeffekt des Schalenelementes in dem Knochen ergibt.

Die länglichen Öffnungen sind nach einer weiteren Ausgestaltung der Erfindung schräg zur Gewindeachse, vorzugsweise unter einem Winkel von 30°, in die Außenschale gefräst. Der angegebene Winkelwert ist nicht einschränkend zu verstehen; er kann nach oben oder unten variieren.

Zur Verbesserung des Einschraubmomentes nimmt die Höhe und die Länge der in Reihen winklig zur Schalenachse angeordneten Gewindegangabschnitte vom proximalen Ende der Außenschale zum distalen Ende hin zu. Dieser Ausgestaltung der Erfindung kommt in Kombination mit der erfindungsgemäßen Anordnung der länglichen Öffnungen sowie mit der doppelgängigen Auslegung des Schneidgewindes eine besondere Bedeutung zu, da es sich herausgestellt hat, daß diese Kombination das Einschrauben des Schalenelementes wesentlich erleichtert und das Schalenelement nach Implantation der Prothese sicher und dauernd befestigt ist.

Außen- und Innenschale können einteilig gegossen werden. Alternativ werden beide Schalenteile jeweils getrennt gegossen, die Innenschale passend in die Außenschale gesteckt und beide Schalenteile zum Beispiel durch Kaltschweißung verbunden.

Bevorzugt wird nach einer weiteren Ausgestaltung der Erfindung eine konische Form der Außen- und Innenschale, wobei zum Einschrauben des Schalenelementes in den Hüftknochen an der Innenseite der Innenschale eine Nut ausgebildet ist, an der ein Werkzeug angreifen kann. Nach erfolgter Einschraubung wird ein passend geformtes Inlay in die Innenschale eingesetzt, welches über Vorsprünge mit der oben erwähnten Nut der Innenschale zusammenwirkt, wodurch eine effektive Verriegelung des Inlays in der Innenschale gegeben ist.

Das aus Außenschale, Innenschale und Inlay zusammengesetzte Schalenelement wird distal von einem radialen, ringförmigen Flansch des Inlays, auf dem die Außenschale und Innenschale sich abstützen, und proximal von den annähernd auf einer Höhe liegenden, proximalen Endflächen der Außenschale, Innenschale und Inlay begrenzt.

Ein Ausführungsbeispiel wird nachfolgend anhand von Zeichnungen näher erläutert. Es zeigen:
- Fig. 1: einen Querschnitt durch eine Außenschale eines Schalenelementes nach der Erfindung,
- Fig. 2: eine Seitenansicht der Außenschale nach Fig. 1,
- Fig. 3: eine Draufsicht auf die Außenschale nach Fig. 1,
- Fig. 4: einen Querschnitt durch eine Innenschale eines Schalenelementes nach der Erfindung,
- Fig. 5: einen Querschnitt durch ein Inlay eines erfindungsgemäßen Schalenelementes,
- Fig. 6: eine Draufsicht auf das Inlay nach Fig. 5,
- Fig. 7: einen Schnitt durch ein aus Außenschale, Innenschale und Inlay zusammengesetztes Schalenelement nach der Erfindung.

Das Schalenelement 1 besteht aus einer in einem Hüftknochen zu implantierenden Außenschale 10 und einer gelenkseitigen Innenschale 20, in die ein Inlay eingesetzt wird.

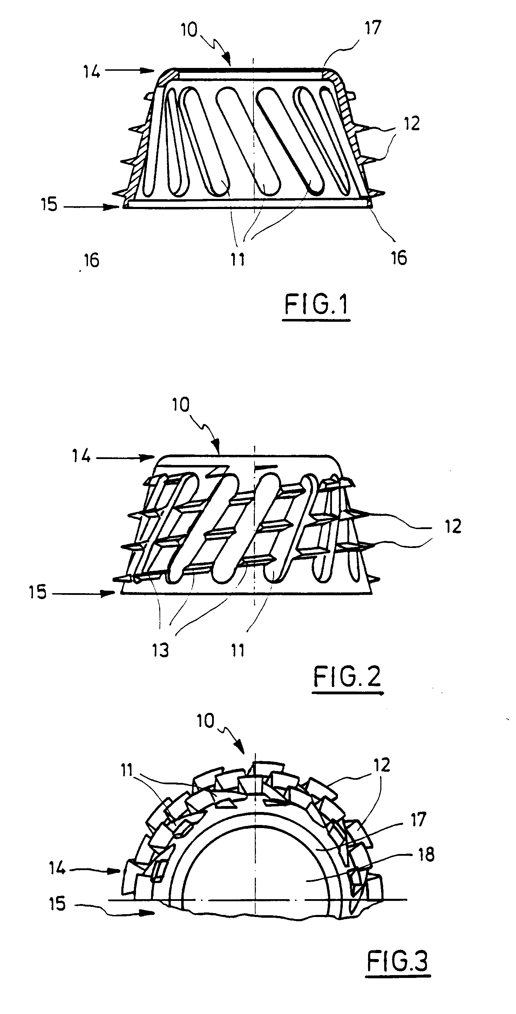

Die konisch geformte Außenschale 10 des Schalenelementes 1 weist im wesentlichen quer zur Umfangsrichtung, mehrere, über die Fläche der Außenschale verteilte, längliche Öffnungen 11 auf, die sich winklig zur Achse des Schalenelements, nahezu über die Höhe der Außenschale 10 erstrecken. Gemäß Fig. 1 sind die Öffnungen 11 unter einem Winkel von 30° zur Achse und in einem Winkel von 10° in Drehrichtung geneigt, d.h. in einem Winkel von 10° zum Radius der Außenschale 10 in die Außenschale 10 gefräst. Der letztere Winkel kann auch einige Grade kleiner oder größer sein. Der Winkel der Öffnungen 11 zur Achse kann generall im Bereich von 20 bis 40° liegen. Auf der dem Hüftknochen zugewandten Seite der Außenschale sind Gewindegangabschnitte 12 ausgebildet. Nach Fig. 2 sind die Abschnitte 12 in Reihen 13 winklig zur Achse und im Winkel zu den länglichen Öffnungen 11 angeordnet. Die Gewindegangabschnitte 12 bilden zusammen ein doppelgängiges Gewinde, das ein Einschrauben in den Hüftknochen mit relativ kleinem Drehmoment ermöglicht. Gleichzeitig bewirkt es einen sicheren Sitz der Hüftpfanne. Wie insbesondere in Fig. 3 zu erkennen ist, nimmt sowohl die Höhe wie auch die Länge der Gewindegangabschnitte 12 vom proximalen Ende 14 der Außenschale 10 zum distalen Ende 15 hin zu. Die zwischen den Gewindegangabschnittsreihen 13 sich erstreckenden länglichen Öffnungen 11 wirken wie die Nut eines Gewindeschneiders. Am distalen Ende 15 der Außenschale ist an deren Innenseite eine Umfangsnut 16 geformt. Das proximale Ende 14 der Außenschale 10 ist außen bei 17 gerundet und läßt eine durch eine kreisförmige Kante 18 gebildete Öffnung 18 frei.

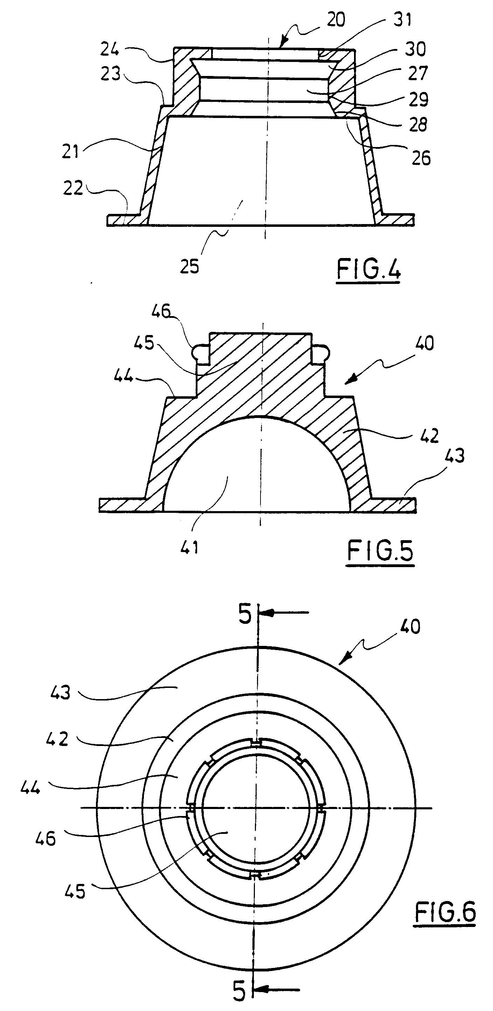

Nach Fig. 4 besteht die Innenschale 20 des Schalenelementes 1 aus einem konischen Abschnitt 21 mit einem distalen radialen Flansch 22 und einem über eine Schulter 23 mit dem Abschnitt 21 verbundenen proximalen zylindrischen Abschnitt 24. Der konische Abschnitt 21 bildet einen konischen Raum 25, der an einer Schulter 26 endet. An die Schulter 26 schließt sich ein konischer Innenabschnitt 28 an, der sich von distal nach proximal radial verengt und der gefolgt wird von einem zylindrischen Abschnitt 29. Angrenzend an den zylindrischen Abschnitt 29 ist eine Nut 30 gebildet, die von dem Ringrand 31 einer Öffnung begrenzt wird.

Das Inlay 40 weist nach Fig. 5 eine halbkugelige Ausnehmung 41 zur Aufnahme einer typischen Gelenkkugel auf. Die Ausnehmung 41 wird von den Innenflächen eines konischen Abschnitts 42 des Inlays geformt. Ein radialer Flansch 43 ist am distalen Ende des konischen Abschnitts 42 gebildet. Ausgehend von einer Schulter 44 des konischen Abschnitts 42 erstreckt sich ein im wesentlichen zylindrischer Abschnitt 45, an dessen Außenseite insgesamt sechs Vorsprünge 46 geformt sind, die gemäß Fig. 6 in Umfangsrichtung beabstandet sind.

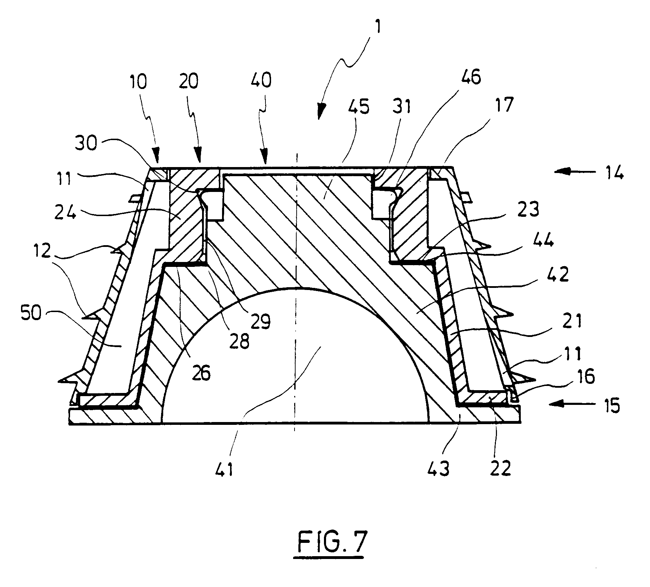

In Fig. 7 ist die Innenschale 20 in die Außenschale 10 eingesetzt. Der Flansch 22 der Innenschale wird von der Umfangsnut 16 der Außenschale aufgenommen, während der zylindrische Abschnitt 24 der Innenschale passend in die Öffnung 18 der Außenschale eingreift. Zwischen Außen- und Innenschale 10, 20, die einteilig geformt oder miteinander verschweißt sind, ist ein ringförmiger, konischer Zwischenraum 50 gebildet, in den vor dem Einsetzen des Schalenelementes 1 in den Hüftknochen Knochensubstanz durch die länglichen Öffnungen 11 eingelagert werden kann. Zum Einschrauben des aus Außen- und Innenschale zusammengesetzten Schalenelementes greift ein Werkzeug in die Nut 30 der Innenschale ein. Durch das aus den Gewindegangabschnitten 12 und den länglichen Öffnungen 11 gebildete Schneidgewinde läßt sich das Schalenelement 1 mit Hilfe eines nicht gezeigten Werkzeugs in den Hüftknochen einschrauben und verankern.

Beim Einsetzen des Inlays 40 in die Innenschale 20, das z.B. aus P.E. Besteht und nachgebend ist, verformen sich die Vorsprünge 46 vorübergehend an dem zylindrischen Abschnitt 29 an der Innenseite der Innenschale, indem sie abgebogen und zur Seite gepreßt werden in die Abstände dazwischen bis sie in die Nut 30 eingreifen und so das Inlay in der Innenschale verriegeln. Die Schulter 44 des Inlays tritt in Eingriff mit der Schulter 26 der Innenschale und auf dem radialen Flansch 43 des Inlays liegt die Unterseite des radialen Flansches 22 der Innenschale an sowie nach außen der distale Endabschnitt der Außenschale 10.

Der zylindrische Abschnitt 45 des Inlays liegt in dem Durchgang 27 der Innenschale und ist ringförmig von dem zylindrischen Abschnitt 24 der Innenschale umgeben, der in die Öffnungskante 18 der Außenschale passend eingreift, so daß die Öffnung 18 von den proximalen Endflächen des Inlays und der Innenschale annähernd auf Höhe der Außenseite der Außenschale abgedeckt wird.

Nach Implantation einer Gelenkendoprothese kann in den Zwischenraum 50 Hüftknochenmaterial über die Öffnungen 11 einwachsen und mit der dort vor der Implantation eingebrachten Knochensubstanz zusammenwachsen und somit das Schalenelement fest im Hüftknochen verankern.

## Patentansprüche

1. Schalenelement (1) zur Aufnahme einer Hüftgelenkendoprothese, mit einer dem Hüftknochen zugewandten Außenschale (10) und einer dazu radial beabstandeten Innenschale (20), die auf ihrer Gelenkseite ein Inlay (40) für eine Gelenkkugel der Endoprothese aufnimmt, wobei auf der dem Hüftknochen zugewandten Seite der Außenschale (10) radial vorstehende, in Umfangsrichtung verlaufende Gewindegangabschnitte (12) geformt sind, die in Reihen (13) angeordnet und von winklig zur Gewindeachse und im wesentlichen quer zu den Gewindegangabschnitten (12) verlaufenden Nuten unterbrochen sind, wodurch ein Schneidgewinde geformt ist zum Einschrauben in den Hüftknochen; und mit mehreren in der Außenschale (10) geformten, über die Fläche der Außenschale verteilten Durchbrüchen in Form von länglichen Öffnungen (11), die einen zwischen der Innenschale (20) und der Außenschale (10) gebildeten Zwischenraum (50) mit dem an der Außenschale anliegenden Hüftknochen verbinden, über die in den Zwischenraum Knochensubstanz eingebracht werden kann, und die nach erfolgter Implantation eine Versorgung der eingebrachten Knochensubstanz sowie ein Hineinwachsen von neuer Hüftknochensubstanz ermöglichen, dadurch gekennzeichnet, daß die Nuten über die gesamte in Umfangsrichtung der Außenschale (10) sich erstreckende Nutbreite und annähernd über die Höhe der Außenschale (10) als längliche Öffnungen (11) ausgebildet sind und die länglichen Öffnungen (11) in einem Winkel zum Radius der Außenschale (10) zur Drehrichtung geneigt ausgebildet sind, vorzugsweise gefräst und vorzugsweise unter einem Winkel von 10°.

2. Schalenelement nach Anspruch 1, dadurch gekennzeichnet, daß das Schneidgewinde doppelgängig ist.

3. Schalenelement nach Anspruch 1 oder 2, dadurch gekennzeichnet, daß die länglichen Öffnungen (11) in der Außenschale (10) schräg zu der Gewindeachse angeordnet sind, vorzugsweise unter einem Winkel von 30°.

4. Schalenelement nach einem der Ansprüche 1 bis 3, dadurch gekennzeichnet, daß die Höhe der Gewindegangabschnitte (12) vom proximalen Ende (14) der Außenschale (10) zum distalen Ende (15) hin zunimmt.

5. Schalenelement nach einem der Ansprüche 1 bis 4, dadurch gekennzeichnet, daß die Länge der Gewindegangabschnitte schnitte (12) vom proximalen Ende (14) der Außenschale (10) zum distalen Ende (15) hin zunimmt.

6. Schalenelement nach einem der Ansprüche 1 bis 5, dadurch gekennzeichnet, daß das Schalenelement (1) einteilig gegossen ist.

7. Schalenelement nach einem der Ansprüche 1 bis 5, dadurch gekennzeichnet, daß die Außenschale (10) und die Innenschale (20) jeweils einteilig gegossen sind, die Innenschale passend in die Außenschale unter Bildung des Zwischenraumes (50) gesteckt ist und beide Schalenteile, z.B. durch Kaltschweißung, miteinander verbunden sind.

8. Schalenelement nach einem der Ansprüche 1 bis 7, dadurch gekennzeichnet, daß die Außenschale (10) und die Innenschale (20) konisch geformt sind.

9. Schalenelement nach Anspruch 7 und 8, dadurch gekennzeichnet, daß die Innenschale (20) aus einem konischen Abschnitt (21) mit einem radialen Flansch (22) und einem zylindrischen Abschnitt (24) besteht, wobei der zylindrische Abschnitt (24) in eine Öffnung (18) der Außenschale (10) eingreift und der radiale Flansch (22) in Eingriff mit einer am distalen Ende (15) der Außenschale angeordneten Umfangsnut (16) tritt.

10. Schalenelement nach Anspruch 9, dadurch gekennzeichnet, daß an der Innenfläche des zylindrischen Abschnitts (24) der Innenschale (20) eine Nut (30) geformt ist zur Aufnahme eines Werkzeugs zum Einschrauben des Schalenelementes (1) in den Hüftknochen.

11. Schalenelement nach Anspruch 8, dadurch gekennzeichnet, daß das Inlay (40) aus einem konischen Abschnitt (42) mit einem radialen Flansch (43) und einem im wesentlichen zylindrischen Abschnitt (45) besteht, wobei nach Einschrauben des aus Außen- und Innenschale zusammengesetzten Schalenelementes und beim Einsetzen des Inlays in das Schalenelement der radiale Flansch (43) des Inlays (40) sich gegen die Unterseite des radialen Flansches (22) der Innenschale (20) anlehnt.

12. Schalenelement nach Anspruch 11, dadurch gekennzeichnet, daß an dem im wesentlichen zylindrischen Abschnitt (45) des Inlays (40) mehrere, in Umfangsrichtung beabstandete Vorsprünge (46) ausgebildet sind, die an der Nut (30) des zylindrischen Abschnitts (24) der Innenschale (20) angreifen und das Inlay (40) in der Innenschale verriegeln.

## Claims

1. A cup element (1) for receiving a hip joint endoprosthesis, comprising an outer shell (10) facing the hip bone and an inner shell (20) radially spaced therefrom which inner shell receives an inlay (40) for a joint ball of the endoprosthesis at the side facing the joint, wherein the surface of the outer shell (10) facing the hip bone is provided with a plurality of radially projecting peripherally extending thread portions (12) shaped in rows (13) which are interrupted by grooves extending under an angle with respect to the thread axis and substantially perpendicular to said thread portions (12), thereby defining a cutting thread for screwing into the hip bone, and comprising a plurality of recesses in the outer shell (10) spaced across the surface of the outer shell, said recesses being defined by elongate openings (11) communicating an intermediate space (50) defined between said inner shell (20) and said outer shell (10) with the hip bone engaging the outer shell, through which openings bone substance may be introduced into the intermediate space, and which openings allow a treatment of the bone substance introduced after the implantation has been completed as well as enhancing the growth of new bone tissue, characterized in that the grooves are defined to be elongate openings (11) extending over the full width of the groove in the periphery of the outer shell (20) and extending substantially across the height of the outer shell (10) and that the elongate openings (11) are designed to slope with respect to the direction of rotation under an angle with respect to the radius of the outer shell (10), that the openings are preferably milled and that the angle is preferably 10°.

2. The cup element of claim 1, characterized in that the cutting thread is a double-thread.

3. The cup element of claim 1 or 2, characterized in that the elongate openings (11) in the outer shell (10) slope with respect to the thread axis, preferably under an angle of 30°.

4. The cup element of one of claims 1 to 3, characterized in that the height of the thread portions (12) increases from the proximal end (14) to the distal end (15) of the outer shell (10).

5. The cup element of one of claims 1 to 4, characterized in that the length of the thread portions (12) increases from the proximal end (14) of the outer shell (10) to the distal end (15).

6. The cup element of one of claims 1 to 5, characterized in that the cup element (1) is integrally casted.

7. The cup element of one of claims 1 to 5, characterized in that the outer shell (10) and the inner shell (20) each are integrally casted, that the inner shell fits into the outer shell forming an intermediate space (50) therebetween and that both shell portions are connected to each other by cold welding, for example.

8. The cup element of one of claims 1 to 7, characterized in that the outer shell (10) and the inner shell (20) are conically formed.

9. The cup element of claim 7 and claim 8, characterized in that the inner shell (20) comprises a conical portion (21) including a radial flange (22) and a cylindrical portion (24), wherein the cylindrical portion (24) engages an aperture (18) of the outer shell (10) and that the radial flange (22) engages a peripheral groove (16) provided at the distal end (15) of the outer shell.

10. The cup element of claim 9, characterized in that a groove (30) is formed at the inner surface of the cylindrical portion (24) of the inner shell (20), said groove being engaged by a tool for screwing the cup element (1) into the hip bone.

11. The cup element of claim 8, characterized in that the inlay (40) comprises a conical portion (42) including a radial flange (43) and a substantially cylindrical portion (45), wherein after screwing the cup element composed of the outer shell and the inner shell and while inserting the inlay into the cup element the radial flange (43) of the inlay (40) engages the lower side of the radial flange (22) of the inner shell (20).

12. The cup element of claim 11, characterized in that the substantially cylindrical portion (45) of the inlay (40) includes a plurality of peripherally spaced projections (46) engaging the groove (30) of the cylindrical portion (24) of the inner shell (20), thus locking the inlay (40) to the inner shell.

## Revendications

1. Elément formant coque (1) destiné à recevoir une endoprothèse de l'articulation de la hanche, comportant une coque extérieure (10) orientée vers l'os iliaque et, à distance radiale de ladite coque extérieure, une coque intérieure (20), qui reçoit côté articulation un insert (40) destiné à la rotule d'articulation de l'endoprothèse,
dans lequel des sections de filetage (12) qui s'étendent dans le sens périphérique, faisant saillie radialement, sont formés sur le côté de la coque extérieure (10) orienté vers l'os iliaque, sont disposés en série (13), sont interrompus par des rainures orientées sensiblement à la perpendiculaire des sections de filetage (12) et forment un angle par rapport à l'axe du filetage, ce qui forme un filetage sectionné permettant le vissage dans l'os iliaque ;
l'élément comportant, formés dans la coque extérieure (10) et répartis sur sa superficie, plusieurs passages en forme d'ouvertures oblongues (11) qui relient un espace intermédiaire (50), formé entre la coque intérieure (20) et la coque extérieure (10) avec l'os iliaque qui est au contact de la coque extérieure, par lesquelles il est possible d'introduire de la substance osseuse dans cet espace intermédiaire, et qui une fois l'implantation effectuée, permettent l'alimentation de la substance osseuse introduite, ainsi que la croissance de nouvelle substance de l'os iliaque,
caractérisé en ce que sur la totalité de la largeur rainurée s'étendant dans le sens périphérique de la coque extérieure (10) et sur la quasi totalité de la hauteur de coque extérieure (10), les rainures sont conformées en ouvertures oblongues (11),
et en ce que les ouvertures oblongues (11) sont inclinées dans le sens de rotation, forment un angle par rapport au rayon de la coque extérieure (10), sont de préférence fraisées et forment de préférence un angle de 10°.

2. Elément formant coque selon la revendication 1, caractérisé en ce que le filetage sectionné est double.

3. Elément formant coque selon la revendication 1 ou 2, caractérisé en ce que, dans la coque extérieure, les ouvertures oblongues (11) sont disposées obliquement par rapport à l'axe du filetage, de préférence en formant un angle de 30°.

4. Elément formant coque selon l'une des revendications 1 à 3, caractérisé en ce que la hauteur des sections de filetage (12) augmente de l'extrémité proximale (14) à l'extrémité distale (15) de la coque extérieure (10).

5. Elément formant coque selon l'une des revendications 1 à 4, caractérisé en ce que la longueur des sections de filetage (12) augmente de l'extrémité proximale (14) à l'extrémité distale (15) de la coque extérieure.

6. Elément formant coque, selon l'une des revendications 1 à 5, caractérisé en ce que l'élément formant coque (1) est moulé d'un seul tenant.

7. Elément formant coque selon l'une des revendications 1 à 5, caractérisé en ce que la coque extérieure (10) et la coque intérieure (20) sont chacune moulées d'un seul tenant, la coque intérieure étant enfoncée de façon ajustée dans la coque extérieure en formant l'espace intermédiaire (50) et les deux parties de coque étant liées l'une à l'autre, par exemple par soudage à froid.

8. Elément formant coque selon l'une des revendications 1 à 7, caractérisé en ce que la coque extérieure (10) et la coque intérieure (20) sont de forme conique.

9. Elément formant coque selon les revendications 7 et 8, caractérisé en ce que la coque intérieure (20) est constituée d'une section conique (21) avec une bride radiale (22), et d'une section cylindrique (24), la section cylindrique (24) pénétrant dans une ouverture (18) de la coque extérieure, et la bride radiale (22) venant en prise avec un rainure périphérique (16) prévue à l'extrémité distale (15) de la coque extérieure.

10. Elément formant coque selon la revendication 9, caractérisé en ce qu'une rainure (30) est formée sur la surface intérieure de la section cylindrique (24) de la coque intérieure (20), pour recevoir un outil destiné à visser l'élément formant coque (1) dans l'os iliaque.

11. Elément formant coque selon la revendication 8, caractérisé en ce que l'insert (40) est constitué d'une section conique (42) avec une bride radiale (43), et d'une section (45) sensiblement cylindrique, la bride radiale (43) de l'insert (40) venant en appui contre le côté inférieur de la bride radiale (22) de la coque intérieure (20) après le vissage de l'élément formant coque constitué des coques extérieure et intérieure, et lors de la mise en place de l'insert dans l'élément formant coque.

12. Elément formant coque selon la revendication 11, caractérisé en ce que plusieurs saillants (46) espacés dans le sens périphérique, qui viennent en prise avec la rainure (30) de la section cylindrique (24) de la coque intérieure (20) et qui verrouillent l'insert (40) dans la coque intérieure, sont formés dans la section sensiblement cylindrique (45) de l'insert (40).
